(19) Europäisches Patentamt
European Patent Office
Office européen des brevets

(11) Numéro de publication: **0 138 715**
**B1**

(12) **FASCICULE DE BREVET EUROPEEN**

(45) Date de publication du fascicule du brevet: **01.07.87**

(51) Int. Cl.⁴: **C 07 C 103/44,** A 61 K 31/16

(21) Numéro de dépôt: **84402066.9**

(22) Date de dépôt: **12.10.84**

# (54) Dérivés de N-(4-acétylaminophénacyl)-amine, leur utilisation en thérapeutique et leur procédé de préparation.

(30) Priorité: **14.10.83 FR 8316405**

(43) Date de publication de la demande:
**24.04.85 Bulletin 85/17**

(45) Mention de la délivrance du brevet:
**01.07.87 Bulletin 87/27**

(84) Etats contractants désignés:
**AT BE CH DE FR GB IT LI LU NL SE**

(56) Documents cités:
**CH-A- 348 240**
**US-A-3 876 786**

**IL FARMACO, Edizione Scientifica, vol. 23, no. 3, mars 1968, pages 221-226; L. VILLA et al.: "Ricerche chimico-fisiche sulle catecolamine. Nota IV - Costanti di ionizzazione di feniletanolamine meta e para sostituite"**

(73) Titulaire: **LABORATOIRE L. LAFON Société anonyme dite:**
**1 rue Georges Médéric**
**F-94701 Maisons-Alfort (FR)**

(72) Inventeur: **Lafon, Louis**
**5 rue de l'Alboni**
**F-75016 Paris (FR)**

(74) Mandataire: **Clisci, Serge et al**
**S.A. FEDIT-LORIOT CONSEILS EN PROPRIETE INDUSTRIELLE 38, avenue Hoche**
**F-75008 Paris (FR)**

**EP 0 138 715 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

La présente invention concerne de nouveaux dérivés de N-(4-acétylaminophénacyl)-amine en tant que produits industriels. Elle concerne également leur utilisation en thérapeutique, notamment en tant qu'agents antidépresseurs et pyschostimulants, ainsi que leur procédé de préparation.

On sait que l'on a déjà décrit des dérivés de phénacylamine et étudié leurs propriétés thérapeutiques. On connaît, en particulier de US—A—3 895 030, les N-(2,4,6-trihydroxyphénacyl)-isopropylamine et N-(2,4-6-triméthoxyphénacyl)-pipérazine qui ont été proposées en tant qu'agents vasodilatateurs périphériques, et de FR—A—2 503 143, les N-(2,4,6-triméthoxyphénacyl)-isopropylamine et N-(2,4,6-triméthoxyphénacyl)-tetiobutylamine qui ont été préconisées en tant qu'agents sédatifs, antiagressifs et antidépresseurs du système nerveux central (SNC).

On vient de trouver de façon surprenante que, par rapport aux dérivés de phénacylamine précités, les composés selon l'invention, qui sont structurellement différents, sont plus intéressants en thérapeutique, notamment en tant qu'agents antidépresseurs du SNC. En particulier le chlorhydrate de N-(4-acétylaminophénacyl)-isopropylamine (CRL 41 021) selon l'invention est moins toxique (DL—50 = 1800 mg/kg, per os, chez le rat mâle) et plus actif en tant qu'agent antidépresseur (DOT — dose quotidinne de traitement chez l'homme pour avoir l'effet antidépressure — de 3 mg) que le chlorhydrate de N-(2,4,6-triméthoxyphénacyl)-isopropylamine antérieurement connu (DL—50 = 450 mg/kg, per os, chez le rat mâle; DQT = 30 mg).

Les nouveaux dérivés de phénacylamine selon l'invention sont caractérisés en ce qu'ils sont choisis parmi l'ensemble constitué par

(i) les N-(4-acétylaminophénacyl)-alkylamines de formule générale

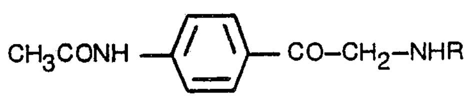

(I)

où R est un groupe $CH(CH_3)_2$ ou $C(CH_3)_3$; et

(ii) leurs sels d'addition.

Par sels d'addition, on entend ici, d'une part, les sels d'addition d'acide obtenus, par réaction de la base libre de formule I avec des acides minéraux ou organiques, et, d'autre part, les sels d'ammonium. Parmi les acides utilisables pour salifier la base de formule I, on peut notamment citer les acides chlorhydrique, bromhydrique, acétique, formique, propionique, oxalique, fumarique, maléique, succinique, benzoïque, cinnamique, mandélique, citrique, malique, tartique, aspartique, glutamique, méthanesulfonique, p-toluènesulfonique. Parmi les composés permettant d'obtenir des sels d'ammonium, on peut notamment citer $ICH_3$ et $ClCH_3$. D'une manière générale les sels d'addition d'acide sont préférés aux sels d'ammonium.

Un certain nombre de composés selon l'invention a été consigné dans le tableua I ci-après.

TABLEAU I

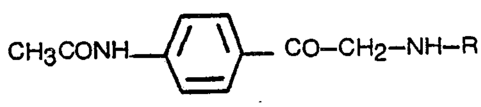

| Produit | N° de code | R |
|---|---|---|
| Ex 1 (a) | CRL 41 021 | $CH(CH_3)_2$ |
| Ex 2 (a) | — | $C(CH_3)_3$ |
| Ex 3 (b) | — | $CH(CH_3)_2$ |
| Ex 4. (c) | — | $CH(CH_3)_2$ |
| Notes<br>(a): chlorhydrates<br>(b): fumarate<br>(c): méthanesulfonate | | |

Les composés selon l'invention peuvent être préparés selon une méthode connue en soi par application de mécanismes réactionnels classiques. Le procédé que l'on préconise ici consiste à faire réagir un halogénure de 4-acétylaminophénacyle de formule

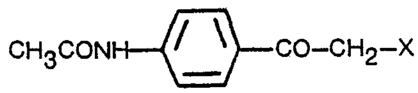

(II)

où X est Cl ou Br, avec une amine primaire de formule

$$H_2NR \qquad (III)$$

où R est défini comme indiqué ci-dessus.

Cette réaction est mise en oeuvre en faisant réagir une mole d'halogénure II avec plus d'une mole d'amine III. De façon avantageuse on fera réagir 1 mole de II avec 1,5 à 5 moles de III dans un solvant inerte, pendant au moins 1 h à une température comprise entre 15 et 40°C.

Les composés de formule I et leurs sels sont utiles en neuropsychopharmacologie en raison de leurs propriétés antidépressives et psycostimulantes. Selon l'invention on préceonise une composition thérapeutique qui est caractérisée en ce qu'elle renferme, en association avec un excipient physiologiquement acceptable, au moins un composé de formule I ou l'une de ses sels d'addition non toxiques, en tant que principe actif.

Bien entendu, dans une telle composition le principe actif intervient en quantité pharmaceutiquement efficace.

Preparation I

Obtention du chlorhydrate de N-(4-acétylaminophénacyl)-isopropylamine

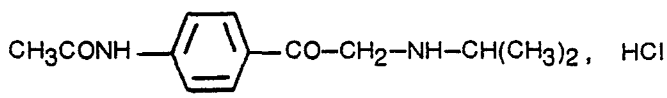

(Exemple 1; N° de code: CRL 41 021)

Dans un réacteur on introduit 21 g (0,1 mole) de chlorure de 4-acétylaminophénacyle dans un mélange comprenant 100 ml de dioxanne, 26 ml (0,3 mole) d'isopropylamine et 20 ml d'eau. On maintient le milieu réactionnel sous agitation à 30—40°C pendant 2 heures. Après addition de 500 ml d'eau glacée, on recueille un précipité qu'on lave et sèche. Par recristallisation dans l'acétate d'éthyle on obtient 21 g de N-(4-acétylaminophénacyl)-isopropylamine qui se présente sous la forme d'une poudre blanche. $F_{inst}$ = 156°C.

Le 21 g de base libre ainsi obtenue sont dissous dans 200 ml d'acétone. Après précipitation au moyen d'un courant gazeux d'HCl, on obtient 20 g de CRL 410 021 (rendement: 73,9% par rapport au chlorure de 4-acétylaminophénacyle) sous forme de poudre blanche $F_{inst} > 250$°C.

Preparation II

Obtention du chlorhydrate de N-(4-acétylaminophénacyl)-tertiobutylamine (Exemple 2)

En procédant comme indiqué dans la Préparation I en remplaçant l'isopropylamine par la tertiobutylamine, on obtient le chlorhydrate de N-(4-acétylaminophénacyl)-tertiobutylamine.

Preparation III

Obtention du méthanesulfonate de N-(4-acétylaminophénacyl)-isopropylamine (Exemple 4)

On fait réagir la N-(4-acétylaminophénacyl)-isopropylamine obtenue selon la Préparation I avec l'acide méthanesulfonique, pour obtenir le méthanesulfonate de N-(4-acétylaminophénacyl)-isopropylamine.

On a résumé ci-après les résultats des essais qui ont été entrepris avec le produit de l'exemple 1, qui est le composé préféré selon l'invention.

A — Toxicite

Chez le rat Sprague Dawley la DL—50 par voie orale est de 1550 ± 54 mg/kg pour les femelles, et de 1800 ± 139 mg/kg pour les mâles.

B — Etude neuropsychopharamacologique

I — Recherche de mouvements stereotypes

Des lots de 6 rats reçoivent une injection intrapéritonéale de CRL 410 021 ou d'eau distillée immédiatement avant d'être placés dans des cages de dimensions réduites où leur comportement stéréotypé est noté toutes les 10 minutes jusqu'à extinction de l'effet. On observe que le CRL 41 021 provoque, dès la dose de 32 mg/kg, l'apparition de mouvements stéréotypés chez le rat. L'intensité de cet effet croît avec la dose, et atteint à 64 mg/kg un niveau comparable à celui obtenu avec 2 mg/kg d'amphétamine.

II — Interaction avec l'apomorphine

*1°) Chez la souris*

Des lots de 6 souris reçoivent le CRL 41 021 par voie intrapéritonéale une demi-heure avant l'injection sous-cutanée de 1 ou 16 mg/kg d'apomorphine. On constate que, aux doses de 64 et 256 mg/kg, le CRL 41 021 exerce un effet hypothermisant intrinsèque et ne modifie pas l'hypothermie, la verticalisation et les stéréotypies induites par l'apomorphine chez la souris.

*2°) Chez le rat*

Le CRL 41 021 est administré par voie intrapéritonéale à des lots de 6 rates une demi-heure avant l'injection sous-cutanée de 0,5 mg/kg d'apomorphine. On constate que, aux doses de 32 et 128 mg/kg, le CRL 41 021 entraîne une potentialisation nette des stéréotypies induites par l'apomorphine chez le rat.

III — Interaction avec l'amphetamine

L'amphétamine (2 mg/kg) est injectée par voie intrapéritonéale à des lots de 6 rats, une demi-heure après l'administration de CRL 41 021 par voie I.P. On constate que, à la dose la plus forte utilisés (128 mg/kg), le CRL 41 021 potentialise nettement les stéréotypies amphétaminiques.

IV — Interaction avec la reserpine

Quatre heures après l'injection intrapéritonéale de 2,5 mg/kg de réserpine, des lots de 12 souris reçoivent le CRL 41 021 par voie I.P. pour étude de l'action de produit sur la température et le ptôsis. On observe que, aux doses de 64 mg/kg et 256 mg/kg, le CRL 41 021 combat nettement l'hypothermie réserpinique alors que le ptôsis induit par la réserpine n'est que faiblement antagonisé à la plus forte dose (256 mg/kg). L'antagonisme de l'hypothermie ne se développe qu'après une latence d'une heure environ.

V — Interaction avec l'oxotremorine

Le CRL 41 021 est administré par voie I.P. à des lots de 6 souris une demi-heure avant l'injection intrapéritonéale de 0,5 mg/kg d'oxotrémorine.

*1°) Action sur la température*

A forte dose (126m mg/kg), le CRL 41 021 exerce un effet hypothermisant et ne combat que faiblement (et dan façon non significative) la baisse de température induite par l'oxotrémorine.

*2°) Action sur les tremblements*

Les tremblements dus à l'oxotrémorine ne sont pas modifiés par le CRL 41 021.

*3°) Action sur les symptômes cholinergiques périphériques*

Le CRL 41 021 ne modifie pas la salivation et la lacrymation mais semble (aux doses de 16, 64 et 256 mg/kg) dimineur l'incidence de la défécation.

VI — Action sur le test des quatre plaques, la traction et l'electrochoc.

Le test est pratiqué sur des lots de 10 souris, une demi-heure après l'administration de CRL 41 021 par voie intrapéritonéale. On observe que le CRL 41 021 n'entraîne pas d'augmentation du nombre de passages punis, qu'il ne provoque pas de déficit moteur majeur et, à forte dose (256 mg/kg), qu'il s'oppose partiellement aux effets convulsivants de l'électrochoc.

VII — Action sur la motilite spontanee

Une demi-heure après avoir reçu par voie intrapéritonéale le CRL 41 021, les souris (12 par dose, 24 témoins) sont placées en actimètre où leur motilité est enregistrée pendant 30 minutes. On constate que le CRL 41 021, aux doses de 16 et 64 mg/kg, entraîne une hypermotilité. Cet effet disparaît à dose plus forte (256 mg/kg).

VIII — Action sur l'aggressive intergroupes

Après avoir séjourné pendant 3 semaines dans chacune des moités d'une cage séparée par une cloison opaque, des groupes de 3 souris reçoivent par voie intrapéritonéale le CRL 41 021. Une demi-heure plus tard, les deux groupes d''une même cage sont réunis par retrait de la cloison et on note le nombre de combats qui surviennent en 10 minutes. On note que, à la dose de 256 mg/kg, le CRL 41 021 diminue le nombre de combats en dépit (ou à cause) d'une excitation observée subjectivement.

IX — Action vis-a-vis de quelques comportements perturbes par divers agents

*1°) Motilité réduite par habituation à l'enceinte*

Après 18 heures de séjour dans les actimètres, les souris (6 par dose, 12 témoins) reçoivent par voie intraéritonéale le CRL 41 021. Elles sont aussitôt replacées dans leurs enceintes respectives et, une demi-heure plus tard, on enregistre leur motilité pendant 30 minutes. On observe que, aux doses de 16m mg/kg, 64 mg/kg et 256 mg/kg, le CRL 41 021 provoque une reprise de l'activité chez la souris habitée à son enceinte.

*2°) Motilité réduite par agression hyproxique*

Une demi-heure après avoir reçu par voie intrapéritonéale le CRL 41 021, les souris (10 par dose, 20 témoins) sont soumises à une anoxie hypoare aigüe [dépression de 600 mmHg (soit environ 8 × $10^4$ pascals) en 90 secondes; détente de 45 secondes], puis elles sont placées en actimètre où leur motilité est enregistrée pendant 10 minutes. On observe que, à la plus forte que a été utilisée (256 mg/kg), le CRL 41 021 entraîne une amélioration sensible de la récupération motrice chez les souris dont la motilité a été déprimée à la suite d'un séjour bref dans une enceinte à pression réduite.

*3°) Anoxie asphyxique*

Des lots de 10 souris reçoivent par voie intrapéritonéale le CRL 41 021 une demi-heure avant l'administration intrapéritonéale de 32 mg/kg de triiodoéthylate de gallamine (agent curarisant de référence). On observe que, à forte dose (256 mg/kg), le CRL 41 021 tend à retarder l'apparition de la mort consécutive à une anoxie asphyxique provoquée par un curarisant.

X — Interaction avec le barbital

Une demi-heure après l'administration par voie intrapéritonéale du CRL 41 021, des lots de 10 souris reçoivent une injection intrapéritonéale de barbital (220 mg/kg). On constate que, dès la dose de 4 mg/kg, le CRL 41 021 diminue la durée du sommeil barbiturique. Cet effet est maximal pour les doses de 64 à 256 mg/kg.

XI — Action sur le "desespoir comportemental"

Une demi-heure après avoir reçu par voie intrapéritonéale le CRL 41 021, des lots de 6 souris mâles sont placés dans un bécher rempli d'eau sur une hauteur de 6 cm. On note la durée totale d'immobilité entre la 2e et la 6e minutes suivant l'immersion. On observe que le CRL 41 021, à la dose de 16 mg/kg, diminue modérément la durée de l'immobilité dite de "désespoir". Cet effet est plus important aux doses de 64 à 256 mg/kg.

XII — Recherche d'une toxicite particuliere chez les souris groupees

Aussitôt après l'administration par voie intrapéritonéale de CRL 41 021, les souris groupées par 10 sont placées dans des cages de petites dimensions. Le nombre d'animaux morts est noté toutes les heures pendant 4 heures puis au bout de 24 heures. La toxicité du CRL 41 021 est déterminée dans le mêmes conditions à raison d'une souris par cage. On observe que le CRL 41 021 n'est pas plus toxique chez les souris isolées que chez les souris groupées.

C — Etude cardio-vasculaire

Le CRL 41 021 a été étudié sur deux types d'animaux:

a) rat normotendu anesthésié,

b) rat normotendu anesthésié, démédullé, atropiné, bivagotomisé.

Le produit a été administré en solution physiologique préparée à froid, par voie intravenieuse (veine jugulaire) sous un volume de 1 ml/kg de poids.

I — Chez la rat anesthesie

L'effet d'une gamme de doses de 0,1 à 30 mg/kg I.V. administrées à 10 minues d'intervalle, a été étudié sur la pression artérielle moyenne et la fréquence cardiaque.

Aux doses étudiées, le CRL 41 021 n'exerce aucune action sur la pression artérielle moyenne ni sur la fréquence cardiaque du rat anesthésié. En revanche, il potentialise la réponse hypertensive à deux doses de noradrénaline (0,5 et 1 µg/kg) administrées après 30 mg/kg, par rapport aux réponses témoins. La bradycardie réflexe est également augmentée en durée et en intensité. On observe par ailleurs que la potentialisation de la résponse à la phényléphrine (1 et 2 µg/kg) est plus douteuse.

II — Chez le rat anesthesie demedulle

*1°) Action sur la pression artérielle moyenne et la fréquence cardiaque*

La même gamme de doses a été étudiée chez la rat anesthésié démédullé que chez la rat non démédullé. On constate que le CRL 41 021 n'exerce aucune action sur la pression artérielle moyenne ni sur la fréquence cardiaque du rat démédullé.

*2°) Action vis-à-vis de la noradrénaline et de la phényléphrine*

Des doses de 3 mg et 30 mg/kg I.V. de CRL 41 021 potentialisent la réponse hypertensive à la noradrénaline et à la phényléphrine, en intensité et en durée. En revanche aucune modification des effets n'est observée sur la fréquence cardiaque des animaux traités à la noradrénaline et à la phényléphrine.

*3°) Action vis-à-vis de l'hypertension due à la stimulation de la moelle à fréquence croissante*

Les doses de 3 et 30 mg/kg de CRL 41 021 sont sans effet sur l'hypertension aux stimulations électriques de la moelle à fréquencés élevées (2 Hz, 5 Hz, 10 Hz) pendant 30 secondes; toutefois, compte tenu de la diminution de la réponse au cours du temps chez les témoins, on peut considérer que les

réponses après 3 et 30 mg/kg de CRL 41 021 sont lègèrement potentialisées.

*4°) Action vis-à-vis de la tachycardie à la stimulation continue de la moelle, à basse fréquence*

Le CRL 41 021 est étudié aux doses de 0,1 à 30 mg/kg I.V. chez 5 rats dont la moelle est stimulée par un courant de faible fréquence (1 Hz) pendant 30 minutes.

La tachycardie due à la stimulation électrique est partiellement antagonisée par l'administration de 30 µg/kg I.V. de clonidine, 5 minutes après le début de la stimulation. Les injections de la gamme de doses de CRL 41 021 sont effectuées toutes les 2 minutes en commençant 5 minutes après la clonidine. On observe que le CRL 41 021 restaure partiellement la tachycardie due à la stimulation et antagonisée par la clonidine, dès la dose de 0,3 mg/kg I.V.

D — Conclusions

Les résultats des essais neuropsychopharmacologiques montrent que le CRL 41 021 est un agent stimulant qui se distingue des stimulants amphétaminiques du fait de son absence de toxicité de groupe, de son antagonisme des hypothermies, et de l'absence de signes de stimulation sympathique.

Les résultats des essais cardiovasculaires mettent en évidence que le CRL 41 021 n'a aucun effet sur la pression artérielle moyenne et la fréquence cardiaque jusqu'à la dose de 30 mg/kg I.V. chez le rat anesthésie démédullé ou non démédullé. La restauration de la tachycardie produite par stimulation continue de la moelle de rat, à basse fréquence, et antagonisée par la clonidine, met en évidence que le CRL 41 021 a une action $\alpha^-$ pré-synaptique; enfin la potentialisation de la phényléphédrine semble suggérer une action sur les récepteurs $\alpha_1$ post-synaptiques.

En clinique, on a obtenu de bons résultats chez l'homme après administration de CRL 41 021, en tant qu'agent antidépresseur sous forme de comprismés ou de gélules renfermant chacun 1 mg de CRL 41 021, à raison de 3 comprimés ou gélules par jour pendant au moins une semaine, dans le traitement de états dépressifs.

**Revendications pour les Etats contractants: BE CH DE FR GB IT LI LU NL SE**

1. Nouveau dérivé de N-(4-acétylaminophénacyl)-amine, utile notamment en thérapeutique, caractérisé en ce qu'il est choisi parmi l'ensemble consitué par:

(i) les N-(4-acétylaminophénacyl)-alkylamines répondnt à la formule generalé:

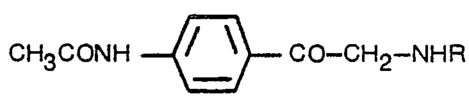

(I)

(où R représente un groupe isopropyle ou tertiobutyle), et

(ii) leurs sels d'addition

2. N-(4-acétylaminophénacyl)-isopropylamine et ses sels d'addition.

3. N-(4-acétylaminophénacyl)-tertiobutylamine et ses sels d'addition.

4. Composition thérapeutique, caractérisé en ce qu'elle renferme, en association avec un excipient physiologiquement acceptable, au moins un dérivé de N-(4-acétylaminophénacyl)-alkylamine de formule (I) selon la revendication 1 ou l'un de ses sels d'addition non toxiques.

5. Procédé de préparation d'un composé de formule (I) selon la revendication 1, caractérisé en ce que l'on fait réagir 1 mole d'halogénure de 4-acétylaminophénacyle de formule:

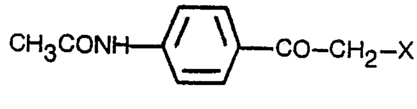

(II)

où X est Cl ou Br, avec plus d'une mole d'une amine de formule

$$H_2NR \qquad (III)$$

où R est isopropyle ou tertiobutyle.

6. Procédé selon la revendication 5, caractérisé en ce que l'on fait réagir 1 mole d'halogénure II avec 1,5 à 5 moles d'amine III pendant au moins 1 h à un température comprise entre 15 et 40°C.

**Revendications pour l'Etat contactant: AT**

1. Procédé de préparation d'un dérivé de N-(4-acétylaminophénancyl)amine, utile notamment en thérapeutique, répondant à la formule générale:

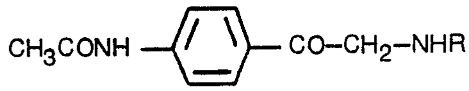

(I)

(où R représente un groupe isopropyle ou tertiobutyle), et ses sels d'addition; ledit procédé étant caractérisé en ce que l'on fait réagir 1 mole d'halogénure de 4-acétylaminophénacyle de formule:

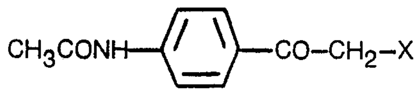

(II)

où X est Cl ou Br, avec plus d'une mole d'une amine de formule

$H_2NR$ (III)

2. Procédé selon la revendication 1, caractérisé en ce que l'on fait réagir 1 mole d'halogénure II avec, 1,5 à 5 moles d'amine III pendant au moins 1 h à une température comprise entre 15 et 40°C.

3. Procédé selon l'une quelconque des revendications 1 et 2, caractérisé en ce que dans la formule II X est Cl, et en ce que dans la formula III le groupe R est isopropyle.

4. Procédé selon l'une quelconque des revendications 1 et 2, caractérisé en ce que dans la formule II X est Cl, et en que dans la formule III le groupe R est tertiobutyle.

**Claims for Contracting States: BE CH DE FR GB IT LI LU NL SE**

1. A N-(4-acetylaminophenyl)amine derivative usefully especially in therapy, which is selected from the group consisting of:

(i) the N-(4-acetylaminophenacyl)alkylamines corresponding to the general formula:

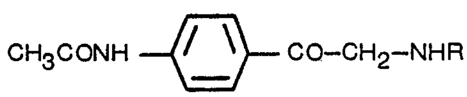

(I)

(in which R represents an isopropyl or tert.-butyl group), and

(ii) their addition salts.

2. N-(4-Acetylaminophenacyl)isopropylamine and its addition salts.

3. N-(4-Acetylaminophenacyl)tert.-butylamine and its addition salts.

4. A therapeutic composition which contains, in association with a physiologically acceptable excipient, at least one N-(4-actylaminophenacyl)alkylamine derivative of the formula (I) as claimed in claim 1, or one of its non-toxic addition salts.

5. A method for the preparation of a compound of the formula (I) as claimed in claim 1, which comprises reacting 1 mol of a 4-acetylaminophenacyl halide of the formula:

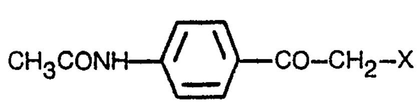

(II)

in which X is Cl or Br, with more than one mol of an amine of the formula

$H_2NR$ (III)

in which R is isopropyl or tert.-butyl.

6. The method as claimed in claim 5, wherein 1 mol of halide II is reacted with 1.5 to 5 mol of amine III, for at least 1 hour, at a temperature of between 15 and 40°C.

**Claims for the Contracting State: AT**

1. A method for preparing a N-(4-acetylaminophenacyl)amine derivative, useful especially in therapy and corresponding to the general formula:

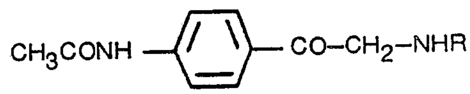 (I)

(in which R represents an isopropyl or tert.-butyl group), and its addition salts, said method comprising reacting 1 mol of a 4-acetylaminophenacyl halide of the formula:

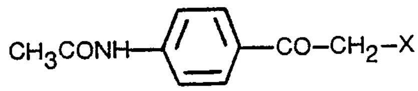 (II)

in which X is Cl or Br, with more than one mole of an amine of the formula

$H_2HR$ (III)

in which R is isopropyl or tert.-butyl.

2. The method as claimed in claim 5, wherein 1 mol of halide II is reacted with 1.5 mol of amine III, for at least 1 hour, at a temperature of between 15 and 40°C.

3. The method according to claim 1 or 2 characterized in that, in the formula II, X is Cl and in that, in the formula III, R is isopropyl.

4. The method according to claim 1 or 2 characterized in that, in the formula II X is Cl and in that, in the formula III, R is tert.-butyl.

**Patentansprüche für Vertragsstaaten: BE CH DE FR GB IT LI LU NL SE**

1. Neue Derivate des N-(4-Acetylamino-phenacyl)-amins, insbesondere zur Verwendung in der Therapie, dadurch gekennzeichnet, daß sie aus der von

(i) N-(4-Acetylaminophenacyl)-alkylaminen der allgemeinen Formel

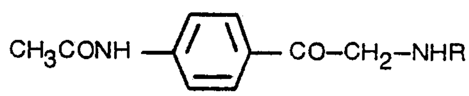 (I)

(in der R eine Isopropyl- oder tertiäre Butylgruppe ist) und

(ii) Additionssalzen derselben gebildeten Gruppe ausgewählt sind.

2. N-(4-Acetylamino-phenacyl)-isopropylamin und seine Additionssalze.

3. N-(4-Acetylamino-phenacyl)-tert.-butylamin und seine Additionssalze.

4. Therapeutische Zusammensetzung, dadurch gekennzeichnet, daß sie zusammen mit einem physiologisch verträglichen Exzipienten mindestens ein Derivat des N-(4-Acetylaminophenacyl)-alkylamins der Formel (I) nach Anspruch 1 oder ein nicht-toxisches Additionssalz desselben enthält.

5. Verfahren zur Herstellung einer Verbindung der Formel (I) nach Anspruch 1, dadurch gekennzeichnet, daß man 1 Mol 4-Acetylamino-phenacyl-halogenid der Formel

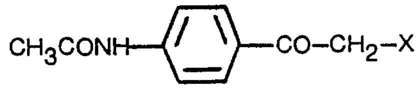 (II)

in der X Cl oder Br ist, mit mindestens einem Mol eines Amins der Formel $H_2NR$ (III), in der R Isopropyl oder tert.-Butyl ist, umsetzt.

6. Verfahren nach Anspruch 5, dadurch gekennzeichnet, daß man 1 Mol des Hologenids II mit 1,5 bis 5 Mol des Amins III während mindestens 1 h bei einer Temperatur zwischen 15 und 40°C umsetzt.

**Patentansprüche für den Vertragsstaat: AT**

1. Verfahren zur Herstellung eines Derivats des N-(4-Acetylamino-phenacyl)-amins, insbesondere zur Verwendung in der Therapie, gemäß der allgemeinen Formel

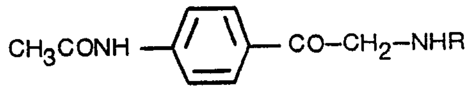

(I)

(in der R eine Isopropyl- oder tert.-Butylgruppe bedeutet) und Additionssalzen desselben, dadurch gekennzeichnet, daß man 1 Mol 4-Acetylamino-phenacyl-halogenid der Formel

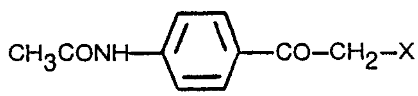

(II)

in der X Cl oder Br ist, mit mehr als einem Mol eines Amins der Formel $H_2NR$ (III), in der R Isopropyl oder tert.-Butyl ist, umsetzt.

2. Verfahren nach Anspruch 1, dadurch gekennzeichnet, daß man 1 Mol Halogenid II mit 1,5 bis 5 Mol eines Amins III mindestens 1 h bei einer Temperatur zwischen 15 und 40°C umsetzt.

3. Verfahren nach einem der Ansprüche 1 oder 2, dadurch gekennzeichnet, daß in der Formel II X = Cl und in der Formel II die Gruppe R Isopropyl ist.

4. Verfahren nach einem der Ansprüche 1 oder 2, dadurch gekennzeichnet, daß in der Formel II X Cl und in der Formel III die Gruppe R tert.-Butyl ist.